Europäisches Patentamt

European Patent Office

Office européen des brevets

(11) Numéro de publication: **0 278 190 B1**

# FASCICULE DE BREVET EUROPEEN

(45) Date de publication de fascicule du brevet: **25.03.92**  (51) Int. Cl.⁵: **C12P 21/02**

(21) Numéro de dépôt: **87402865.7**

(22) Date de dépôt: **15.12.87**

(54) **Procédé de préparation de l'aspartame par voie enzymatique.**

(30) Priorité: **14.01.87 FR 8700323**

(43) Date de publication de la demande:
**17.08.88 Bulletin 88/33**

(45) Mention de la délivrance du brevet:
**25.03.92 Bulletin 92/13**

(84) Etats contractants désignés:
**NL**

(56) Documents cités:
**EP-A- 0 099 585**
**EP-A- 0 124 313**
**EP-A- 0 154 472**

**EUROPEAN JOURNAL OF BIOCHEMISTRY,
vol. 19, no. 1, 1971, pages 51-55; M.J. DESMA-
ZEAUD et al.: "Spécificité de la protéase
neutre de Micrococcus caseolyticus"**

**ANN. BIOL. ANIM. BIOCH. BIOPHYS., vol. 8,
no. 4, 1968, pages 565-577; M. DESMAZEAUD
et al.: "Isolement, purification et propriétés
d'une protéase exocellulaire de Micrococcus caseolyticus"**

(73) Titulaire: **SOCIETE FRANCAISE HOECHST Société anonyme dite:**
**3, avenue du Général de Gaulle**
**F-92800 Puteaux(FR)**

(72) Inventeur: **Paul, François Bernard**
**28 rue du Vivier**
**F-31650 Saint Orens(FR)**
Inventeur: **Monsan, Pierre Frédéric**
**Renoufail Mondoville**
**F-31700 Blagnac(FR)**
Inventeur: **Auriol, Daniel Henri**
**67 avenue de l'U.R.S.S.**
**F-31400 Toulouse(FR)**

(74) Mandataire: **Rinuy, Santarelli**
**14, avenue de la Grande Armée**
**F-75017 Paris(FR)**

Rank Xerox (UK) Business Services

**Description**

La présente invention concerne un procédé de préparation de l'aspartame par voie enzymatique.

L'aspartame ou alpha L-aspartyl L-phénylalaninate de méthyle est un peptide connu (brevet britannique N° 1.042.487) doué de propriétés édulcorantes (R.H. Mazur et al, J. Amer. Chem. Soc., 1969, 61, 2684-91).

On sait l'obtenir soit par voie chimique, soit par voie enzymatique. Les procédés chimiques connus font appel aux méthodes classiques de synthèse peptidique qui exigent la protection temporaire de certains sites réactifs ; de ce fait, ces procédés sont longs, onéreux et peu attrayants. Certains procédés enzymatiques connus autorisent l'accès direct à l'aspartame à partir d'acide L-aspartique et de DL-phénylalaninate de méthyle (demandes de brevet européen N° 74.095, 124.313 et 154.472 et demande de brevet français N° 85.16365). Toutefois, ces procédés présentent une productivité faible peu satisfaisante pour une production industrielle économique.

Or la Demanderesse a découvert avec étonnement un nouveau procédé de préparation de l'aspartame par voie enzymatique obviant à cet inconvénient.

La présente invention a ainsi pour objet un procédé de préparation de l'aspartame par condensation enzymatique directe de l'acide L-aspartique avec le L-phénylalaninate de méthyle caractérisé par le fait qu'il est effectué dans un milieu aqueux homogène contenant en proportions pondérales de 20 à 75 % d'un produit de formule générale I :

$$RO \{CH_2 - CH_2 - O\}_n R \qquad I$$

dans laquelle R représente un groupe méthyle ou éthyle et n représente un nombre entier compris entre 1 et 5, en présence d'une enzyme multimérique possédant une masse moléculaire supérieure à 100.000, ne présentant aucune activité protéolytique, ladite enzyme étant issue d'une culture de micrococcus caseolyticus.

Parmi les produits de formule générale I, l'invention utilise plus particulièrement le diglyme ou le triglyme qui sont respectivement les dénominations communes de l'éther diméthylique du diéthylèneglycol et du tétraoxa-2,5,8,11 dodécane, et avantageusement le triglyme.

Dans des conditions préférentielles de mise en oeuvre du procédé de la présente invention, le procédé est réalisé dans un milieu homogène eau-triglyme contenant en proportions pondérales de 50 à 70 % de triglyme.

La concentration optimale de triglyme dans le milieu réactionnel est celle qui conduit à la meilleure productivité en fonction des différents paramètres de la réaction ; cette concentration optimale se situe le plus souvent aux environs de 60 % en poids de triglyme.

Selon une variante, le procédé objet de la présente invention est aussi caractérisé par le fait qu'il est réalisé en présence de quantités catalytiques d'ions métalliques choisis dans le groupe constitué par les ions zinc, manganèse.

La présence d'un de ces ions métalliques dans le milieu réactionnel de condensation a pour effet d'accélérer très nettement la cinétique réactionnelle sans toutefois modifier le rendement final.

Ces ions métalliques sont apportés dans le milieu réactionnel par l'un de leurs sels hydrosolubles compatibles tels que le chlorure de zinc, le sulfate de zinc, le chlorure de manganèse, le sulfate de manganèse. Leur concentration dans le milieu réactionnel sous forme de sels hydrosolubles compatibles est comprise entre 0,1 mmole et 5 mmoles par litre (soit, exprimée en molarité, entre 0,1 mM et 5 mM).

Avantageusement, l'ion métallique utilisé est l'ion zinc et dans des conditions préférentielles de mise en oeuvre de la variante du procédé objet de la présente invention, le procédé est réalisé en présence d'ions zinc dans un milieu aqueux homogène contenant de 40 à 70 % en poids de triglyme. La concentration optimale d'ions zinc dans le milieu réactionnel est celle qui conduit à la meilleure productivité en fonction de la concentration en triglyme, cette concentration optimale se situe habituellement entre 0,5 et 5 mmoles d'ions zinc par litre.

Le procédé de la présente invention ainsi que sa variante sont mis en oeuvre à une température comprise entre 10°C et environ 50°C, préférentiellement de 15°C à 40°C et à un pH qui peut être compris entre 4 et 7 mais préférentiellement à un pH de 6,4 ± 0,4 (pH qui est déterminé avant l'introduction dans le milieu réactionnel aqueux du produit de formule générale I utilisé).

Le procédé de la présente invention ainsi que sa variante sont mis en oeuvre au départ d'acide L-aspartique et de L-phénylalaninate de méthyle mais on peut utiliser le DL-phénylalaninate de méthyle au lieu du L-phénylalaninate de méthyle. On peut également utiliser le L-aspartate de sodium et le chlorhydrate de L-phénylalaninate de méthyle.

L'agent de condensation de l'acide L-aspartique avec le L-phénylalaninate de méthyle est une enzyme multimérique, possédant une masse moléculaire supérieure à 100.000 issue d'une culture de micrococcus caseolyticus dont une souche a été déposée dans la Collection Nationale de Micro-organismes de l'Institut Pasteur à Paris sous le N° 1.194.

Cette enzyme reconnait l'aspartame ; elle ne possède pas d'activité protéolytique mais elle présente une activité peptidase vis-à-vis de l'aspartame.

On a exprimé son activité par sa capacité d'hydrolyse de l'aspartame. Cette activité est déterminée par la vitesse initiale d'hydrolyse de l'aspartame (qui est linéaire jusqu'à 20 % d'hydrolyse). Une unité, U, correspond à la production d'une micromole par heure de L-phénylalaninate de méthyle à 40°C dans le tampon phosphate de potassium 20 mM à partir d'une solution d'aspartame 17 mM (arrêt par addition de 5 % en poids d'acide chlorhydrique 1N).

La concentration du milieu réactionnel en enzyme influe sur la vitesse de synthèse de l'aspartame durant les premières heures de la réaction. Toutefois, à partir d'une concentration en enzyme, de l'ordre de 300 U par gramme de milieu réactionnel ; la concentration du milieu réactionnel en aspartame est régie par la loi d'action de masse.

Cette loi indique que la réaction de synthèse de l'aspartame ne sera favorisée sur la réaction inverse concurrente d'hydrolyse que si le milieu réactionnel présente une concentration faible en eau ou élevée en acide L-aspartique et en L-phénylalaninate de méthyle. Il est donc avantageux de mettre en oeuvre le procédé de la présente invention ainsi que sa variante avec des concentrations élevées en acide L-aspartique et en L-phénylalaninate de méthyle compatibles avec leur solubilité.

Le déroulement de la réaction peut être suivi par l'analyse de prises d'essais successives avec un analyseur d'amino-acides.

En fin de réaction, la séparation de l'aspartame de son milieu réactionnel peut se réaliser par la mise en oeuvre de procédés connus.

Les exemples suivants sont donnés à titre illustratif et nullement limitatif de la présente invention.

Dans ces exemples, toutes les concentrations sont exprimées par rapport au poids du milieu réactionnel ; la constante K est calculée par la relation :

$$K = \frac{[APM]}{[L-ASP]\,[L-PAM]} M^{-1}$$

dans laquelle APM, L-ASP et L-PAM représentent respectivement l'aspartame, l'acide L-aspartique et le L-phénylalaninate de méthyle.

EXEMPLES

Préparation de l'enzyme

a) Préparation du bouillon de fermentation

Dans un fermenteur, on stérilise à 120°C une solution préparée à partir de 750 litres d'eau, 20 kg de produit connu sous l'appellation de "corn steep" liquide, 20 kg de peptone de caséine, 10 kg d'autolysat de levure et 0,906 kg de chlorure de calcium, solution dont le pH a été ajusté à 7 par addition de lessive de soude. On amène la température du milieu stérile à 30°C puis on introduit 15 litres d'une solution stérile de dextrose à 30 % et 10 litres de bouillon de pied de cuve d'une culture de Micrococcus caseolyticus - (souche déposée à l'Institut Pasteur à Paris sous le ° 1.194).

On laisse fermenter pendant 24 heures à 30°C, sous agitation et air stérile en maintenant le pH constant pendant les 10 premières heures. Après 24 heures, on recueille 1050 litres de bouillon brut de fermentation.

b) Extraction de l'enzyme

On sépare par centrifugation sur centrifugeuse Alfa-Laval type LX les bactéries contenues dans 5000 litres d'un bouillon de culture préparé comme indiqué ci-dessus et l'on recueille 4770 litres de centrifugeat limpide. Au centrifugeat obtenu, on ajoute 2670 kg de sulfate d'ammonium puis, après agitation pendant 30 minutes, 1,2 kg de "Hyflosupercel". On laisse reposer 24 heures puis on élimine le surnageant.

On introduit la fraction insoluble obtenue ci-dessus dans 330 litres d'une solution de chlorure de calcium, on agite 15 minutes et on filtre. On recueille 390 litres d'une solution que l'on concentre par ultra-filtration. Après 12 heures, on obtient 50 litres d'une solution concentrée titrant 50500 unités protéolytiques par $cm^3$. Cette solution est lyophilisée, on obtient ainsi une poudre titrant 580 unités protéolytiques par mg et 1,8 U/mg (hydrolyse de l'aspartame).

L'activité protéolytique est déterminée par mesure (variation de la densité optique à 275 nm) de la quantité de caséine hydrolysée par cette poudre (fraction azotée non précipitable par l'acide trichloroacétique) dans des conditions standard. Une unité d'activité protéolytique est définie comme la quantité de produit qui, agissant dans les conditions du dosage, provoque une variation de la densité optique à 275 nm de 0,001 unité de densité optique par minute par millilitre de solution.

c) Purification de l'enzyme

On dissout 300 g de la poudre précédente dans 10 litres de tampon phosphate de potassium, pH = 6,5, 20 mM contenant 0,1 mM de sulfate de manganèse, puis la solution obtenue est centrifugée durant 30 minutes sous 15.000 gal. afin d'éliminer les insolubles éventuels. La solution limpide obtenue est ensuite soumise à une ultrafiltration dans un module à fibres creuses AMICON équipé d'une cartouche de filtration H 10 P 100 (seuil de coupure 100.000 daltons) commercialisé par la société GRACE, division AMICON, 28231 EPERNON CEDEX. Le rétentat obtenu est soumis à une chromatographie d'échange d'ions sur une colonne DEAE Sepharose Fast Flow K 50 commercialisée par PHARMACIA (section 19 $cm^2$, volume de gel 300 $cm^3$, débit 5 $cm^3$/minute). Après rinçage, on élue par paliers de concentration croissante de chlorure de potassium (200,300 et 400 mM) dans le tampon phosphate de potassium pH = 6,5, 20 mM, contenant 0,1 M de sulfate de manganèse. On suit l'élution par mesure de l'absorption ultraviolette des éluats.

L'enzyme cherchée est éluée dans la fraction correspondant au palier de 200 mM de chlorure de potassium.

A cette étape de purification, l'activité de l'enzyme est de 112 U/mg et elle ne présente plus aucune activité protéolytique.

On recommence un nouveau cycle de purification en refaisant une nouvelle ultrafiltration dans un module à fibres creuses AMICON équipé d'une cartouche de filtration H1 P 100 suivie d'un dessalage sur colonne Sephadex G 25 commercialisée par PHARMACIA (section 19 $cm^2$, volume de gel 1,5 litre, débit 470 $cm^3$/h avec un tampon phosphate de potassium pH = 6,5, 20 mM) et d'une chromatographie d'échange d'ions à pH = 6,0 sur colonne DEAE Sepharose Fast Flow dans les mêmes conditions que précédemment. A cette étape de purification, l'activité de l'enzyme est de 450 U/mg.

Enfin, un troisième cycle de purification effectué par dessalage sur colonne Sephadex G25 suivie successivement d'une filtration sur gel Sephacryl S 200 commercialisé par PHARMACIA (section 19 $cm^2$, volume de gel 1,5 litre, débit 7,5 $cm^3$/h avec tampon phosphate de potassium 50 mM, pH = 6,5 contenant 0,1 M de chlorure de manganèse) et d'une concentration par ultrafiltration permet d'obtenir 65 $cm^3$ de solution enzymatique présentant une activité de 1135 U/mg soit 4000 U/$cm^3$. Cette solution contient 3,26 mg de protéines par $cm^3$ et elle ne présente aucune activité protéolytique sur la caséine.

Une électrophorèse réalisée sur cette enzyme dans des conditions non dénaturantes permet d'observer une bande nette correspondant à une masse moléculaire de 130.000.

EXEMPLES 1-19

Conditions opératoires :
- L-aspartate de sodium : 0,1 M
- chlorhydrate de L-phénylalaninate de méthyle : 0,25 M
- pH = 6,4

Les autres conditions opératoires ainsi que la rendement et la constante K déterminée à l'équilibre réactionnel sont donnés donnés dans le tableau 1 dans lequel :

E représente la concentration du milieu réactionnel en enzyme exprimée en U par gramme de ce milieu;

D représente la concentration du milieu réactionnel en diglyme exprimée en pourcentage pondéral ;

T représente la concentration du milieu réactionnel en triglyme exprimée en pourcentage pondéral ;

N représente la nature de l'ion introduit dans le milieu réactionnel et C représente sa concentration exprimée en mmole par litre ; le sel utilisé est le sulfate de zinc ;

t représente la température en degrés Celsius;

d représente la durée réactionnelle en heures;

4

R représente le rendement en aspartame exprimé en pourcentage de la théorie calculée par rapport à l'acide L-aspartique introduit.

Tableau 1

| N° | E | D | T | N | C | t | d | K | R |
|---|---|---|---|---|---|---|---|---|---|
| 1 | 100 | 0 | 0 | - | 0 | 40 | 2 | 0,03 | 0,74 |
| 2 | 200 | 60 | 0 | - | 0 | 40 | 30 | 0,4 | 8,7 |
| 3 | 200 | 0 | 60 | - | 0 | 40 | 30 | 0,56 | 11,8 |
| 4 | 100 | 0 | 56 | - | 0 | 30 | 30 | 0,28 | 6,4 |
| 5 | 300 | 0 | 56 | - | 0 | 30 | 30 | 0,71 | 14,4 |
| 6 | 500 | 0 | 56 | - | 0 | 30 | 30 | 0,67 | 13,6 |
| 7 | 500 | 0 | 56 | - | 0 | 40 | 30 | 0,68 | 13,7 |
| 8 | 500 | 0 | 61 | - | 0 | 30 | 30 | 0,52 | 10,9 |
| 9 | 500 | 0 | 66 | - | 0 | 30 | 30 | 0,45 | 9,7 |
| 10 | 500 | 0 | 70 | - | 0 | 30 | 30 | 0,22 | 5,2 |
| 11 | 300 | 0 | 56 | Zn | 0,5 | 30 | 6 | 0.70 | 14,2 |
| 12 | 300 | 0 | 56 | Zn | 0,5 | 40 | 6 | 0,75 | 15,0 |
| 13 | 500 | 0 | 56 | Zn | 0,1 | 40 | 6 | 0,73 | 14,6 |
| 14 | 500 | 0 | 56 | Zn | 0,5 | 40 | 6 | 0,73 | 14,8 |
| 15 | 500 | 0 | 56 | Zn | 1,0 | 40 | 6 | 0,73 | 14,7 |
| 16 | 500 | 0 | 56 | Zn | 1,5 | 40 | 6 | 0,73 | 14,7 |
| 17 | 500 | 0 | 56 | - | 0 | 30 | 6 | 0,23 | 4,7 |
| 18 | 500 | 0 | 56 | Zn | 0,5 | 30 | 6 | 0,70 | 14,2 |
| 19 | 300 | 0 | 56 | Zn | 0,5 | 30 | 30 | 0,76 | 15,2 |

Exemples 20-24

Conditions opératoires :
- pH = 6,4
- température : 40°C
- durée : 8 heures
- triglyme 55 % pondéral
- chlorhydrate de L-phénylalaninate de méthyle : 0,5 M
- sulfate de zinc 1 mM
- concentration en enzyme : 1000 U par gramme de milieu.

K et R conservent la signification donnée précédemment.

| N° | Concentration en L-aspartate de sodium | K | R |
|---|---|---|---|
| 20 | 0,025 M | 1,10 | 32 |
| 21 | 0,050 M | 0,68 | 25 |
| 22 | 0,100 M | 0,66 | 24 |
| 23 | 0,150 M | 0,53 | 20 |
| 24 | 0,200 M | 0,42 | 17 |

Exemple 25

On abandonne 4 heures à 40°C, à pH = 6.4 une solution constituée par :
- 155,3 g de chlorhydrate de L-phénylalaninate de méthyle, soit 0.72 mole.
- 15,5 g de L-aspartate de sodium, soit 0. 1 mole,
- 0.068 g de chlorure de zinc,
- 570 g de triglyme,
- Concentration en enzyme : 710 U/cm$^3$ de milieu réactionnel

- et le complément à 1000 g par de l'eau.

A ce stade, l'analyse d'un échantillon du milieu réactionnel indique la présence de 25 mmoles d'aspartame soit un rendement de 25 % de la théorie calculée par rapport au L-aspartate de sodium mis en oeuvre.

**Revendications**

1. Procédé de préparation de l'aspartame par condensation enzymatique directe de l'acide L-aspartique avec le L- phénylalaninate de méthyle caractérisé par le fait qu'il est effectué dans un milieu aqueux homogène contenant en proportions pondérales de 20 à 75% d'un produit de formule générale I :

   $$RO \{CH_2 - CH_2 - O\}_n R \qquad I$$

   dans laquelle R représente un groupe méthyle ou éthyle et n représente un nombre entier compris entre 1 et 5, en présence d'une enzyme multimérique possédant une masse moléculaire supérieure à 100.000, ne présentant aucune activité protéolytique sur la caséine, ladite enzyme étant issue d'une culture de micrococcus caseolyticus.

2. Procédé selon la revendication 1, caractérisé par le fait que le radical R du produit de formule générale I est le radical méthyle.

3. Procédé selon la revendication 2, caractérisé par le fait que le symbole n du produit de formule générale I représente le nombre 2 ou 3.

4. Procédé selon la revendication 3, caractérisé par le fait que le produit de formule générale I est le triglyme.

5. Procédé selon l'une quelconque des revendications 1 à 4, caractérisé par le fait qu'il est réalisé en présence d'ions métalliques choisis dans le groupe constitué par les ions zinc et manganèse.

6. Procédé selon la revendication 5, caractérisé par le fait que l'ion métallique est l'ion zinc.

**Claims**

1. A process for the preparation of aspartame by direct enzymatic condensation of L-aspartic acid with methyl L-phenylalaninate wherein it is carried out in a homogeneous aqueous medium containing 20 to 75 weight percent of a compound of general formula I:

   $$RO -(CH_2 - CH_2 - O)-_n R \qquad I$$

   wherein R represents a methyl or ethyl group and n represents an integer between 1 and 5, in the presence of a multimeric enzyme having a molecular weight greater than 100 000 and exhibiting no proteolytic activity on casein, said enzyme being a product of a culture of micrococcus caseolyticus.

2. A process according to claim 1 wherein the radical R of the compound of general formula I is the methyl radical.

3. A process according to claim 2 wherein the symbol n of the compound of general formula I represents the number 2 or 3.

4. A process according to claim 3 wherein the compound of general formula I is tetraoxa-2,5,8,11-dodecane.

5. A process according to claims 1 to 4 wherein said process is carried out in the presence of metallic ions selected from the group constituted by zinc and manganese ions.

6. A process according to claim 5 wherein the metallic ion is zinc.

**Patentansprüche**

1. Verfahren zur Herstellung von Aspartam durch direkte enzymatische Kondensation von L-Asparaginsäure mit L-Phenylalaninmethylester, dadurch gekennzeichnet, daß das Verfahren in einem homogenen wäßrigen Milieu, das 20 bis 75 Gew.-% eines Produktes der allgemeinen Formel

   $RO-(CH_2-CH_2-O)_n-R$      I

   enthält, worin
   R eine Methyl- oder Ethylgruppe ist und
   n eine ganze Zahl zwischen 1 und 5 ist,
   in Gegenwart eines multimeren Enzyms, dessen Molmasse mehr als 100.000 beträgt, das keine proteolytische Wirkung auf Casein besitzt und das von einer Kultur von Micrococcus caseolyticus stammt, durchgeführt wird.

2. Verfahren gemäß Anspruch 1, dadurch gekennzeichnet, daß das Radikal R des Produktes der allgemeinen Formel I ein Methylradikal ist.

3. Verfahren gemäß Anspruch 2, dadurch gekennzeichnet, daß im Produkt der allgemeinen Formel I das Zeichen n die Zahl 2 oder 3 ist.

4. Verfahren gemäß Anspruch 3, dadurch gekennzeichnet, daß das Produkt der allgemeinen Formel I Triglyme ist.

5. Verfahren gemäß irgendeinem der Ansprüche 1 bis 4, dadurch gekennzeichnet, daß das Verfahren in Gegenwart von Metallionen, ausgewählt aus der aus Zink- und Manganionen bestehenden Gruppe, durchgeführt wird.

6. Verfahren gemäß Anspruch 5, dadurch gekennzeichnet, daß das Metallion ein Zinkion ist.